# EUROPEAN PATENT SPECIFICATION

(11) **EP 1 958 595 B1**
(45) Date of publication and mention of the grant of the patent: **27.01.2010**
(21) Application number: 08250469.7
(22) Date of filing: 07.02.2008
(51) Int. Cl.: A61F 2/30

(54) **Prosthetic implant for use without bone cement**
Prothetisches Implantat zur Verwendung ohne Knochenzement.
Implant prothétique pour une utilisation sans ciment osseux.

(30) Priority: 14.02.2007 GB 0702864
(43) Date of publication of application: 20.08.2008
(73) Proprietor: BENOIST GIRARD SAS, 14201 Hérouville-Saint-Clair Cédex (FR)
(72) Inventor: Jones, Eric, Kildimo, County Limerick (IE)
(74) Representative: Bridge-Butler, Alan James

(56) References cited:
- EP-A- 0 159 036
- EP-A- 0 561 263
- WO-A-00/59410
- WO-A-03/086244
- WO-A-2006/091423
- US-A- 5 443 512
- US-A- 5 489 306

## Description

This invention relates to a collarless prosthetic femoral implant which has an attachment portion for insertion into or attachment to a patient's bone without bone cement.

The term 'bone ongrowth' will be used herein to indicate a surface onto which bone can grow, for example a roughened surface. The term 'bone ingrowth' will be used herein to indicate a surface in which bone can grow inwardly, for example a porous surface.

It is known from, for example, US 5,665,121 to use a sheath made from a synthetic plastics material which could have a roughened outer surface to promote bone ongrowth over the stem of a femoral prosthesis for use without bone cement. It is also known from US 4,650,489 to provide a femoral prosthesis with an outer sheath made of stainless steel or titanium steel which encloses the stem but is separated from it by a layer of elastomeric material such as silicon or a butyl rubber. The inner surface of the metallic sheath and the outer surface of the stem are indented to retain the elastomeric material in place and prevent movement between the stem and the outer metallic sheath. The distal end of the stem is located in a closed cavity filled with an air/gas below the elastomeric material to allow the stem to displace slightly under shock due to the resilience of the elastomeric material but there is no provision to allow the stem to subside downwardly within the sheath after insertion.

The present invention is intended to provide a construction which has the advantage over those referred to in the earlier documents in that it allows a sheath to be proximally loaded by arranging the outer surface to be metallic and encourage bone ingrowth, and for the distal part of the sheath to encourage bone ongrowth or only a very minor ingrowth or none at all. With this arrangement the loading on the distal end of the sleeve is reduced in relation to the proximal loading, which has been found to be desirable.

According to the present invention a collarless prosthetic femoral implant which has an attachment portion in the form of a stem for insertion into or attachment to a patient's bone without bone cement, **characterised in that** the outer surface of said stem is smooth and tapered from the proximal to the distal end, a first layer made of a synthetic resin material extending over said stem and which is not attached thereto to allow subsequent downward movement between the first layer and the smooth stem after fitting and a second layer secured to said first layer and made from a metallic material a first and proximal portion thereof being porous or roughened to encourage bone ingrowth or ongrowth and a second and distal portion thereof being less porous, substantially non-porous, or less roughened in relation to the first portion or smooth.

This type of construction allows for the desirable proximal loading capability.

Thus, the outer surface of the metallic layer can, for example, have an interconnected porosity where bone ingrowth is required on the proximal portion and a roughened surface where bone ongrowth is required on the outer distal surface of the metallic layer or this portion maybe relatively smooth.

In some constructions the required proximal locking can be achieved by a roughened surface on the proximal portion and smooth or less roughened on the distal portion.

The first and second layers can be applied in situ to the attachment portion, for example the plastic sheath is preferably cast into position on the attachment portion with which it is to be used and this can be temporarily fitted with a mechanism to create the distal void.

Alternatively the first and second layers can be preformed as a separate sheath for attachment to the attachment portion. Again, the plastic inner layer can be cast on the intended femoral implant or, alternatively, the inner layer can be moulded into the outer metallic layer.

A sheath can also be made which is not preferentially matched with the intended femoral implant, for example, the sheath could be of a standard dimension which could be used with existing implants.

The second metallic material layer can be made from titanium, titanium alloy or any other suitably bio-compatible metal which contacts directly with the bone..

The metal can be in the form of a preformed shape or can be formed by metallic sputtering or, for example, forming by a laser melting process.

The layer melting process can, for example, be as set forth in US Patent Applications Nos. 20040191106 entitled, "Laser-Produced Porous Surface"; 20060147332 entitled, "Gradient Porous Implant"; 11/295,008 entitled, "Laser-Produced Porous Surface"; and 60/755,260 entitled, "Laser-Produced Implants", the disclosures of which are hereby incorporated by reference herein. As discussed in US Patent Application No.20040191106, the metal structure may be constructed using a selective laser melting or sintering process, which hereby grows the structure in a layer by layer process. In the alternative, the metal structure may be built using an alternate process described in US Patent Application No. 2004019116 wherein the intermediate portion acts as a base or substrate on which the polymer engaging portion and bone ingrowth portion are built thereon, also in a layer-by-layer fashion. Additional techniques for constructing the metal lattice may also be employed such as that disclosed in US Patent Publication No. 20030153981entitled, "Porous Metallic Scaffold for Tissue", the disclosure of which is hereby incorporated by reference herein, as well as additional methods known to those in the art such as that disclosed in Patent Publications Nos. 20060228247 and 20060002810, the disclosures of which are hereby incorporated by reference herein.

Preferably, the synthetic resin material is polymethylmethacrylate (PMMA).

Preferably there is engagement or interlock between the first and second layers, for example by providing a roughened surface on inner surface of the second layer.

The distal end of the first and second layers can be formed as a cup, the inner surface of which is spaced away from the distal end of the attachment portion to provide a distal void when initially located in position to accept subsequent movement between the first layer and the attachment portion after fitting. This movement allows the stem to subside to its natural position after initial weight bearing of the implanted device.

The thickness of the wall formed by the two layers can be between 1 mm and 3 mm and is preferably between 1.8 mm and 2.5 mm.

If desired, the inner surface of the metal second layer can be roughened to enhance the bonding to the outer surface of the synthetic resin first layer. Preferably this bonding can take place with the addition of a PMMA bone cement.

The invention also includes a method of forming the prosthetic implant which has an attachment portion as set forth above, the method includes attaching the first layer of a synthetic resin material by dipping the stem portion into a liquid bath of material or by spraying the material onto the outer surface of the attachment portion, then applying the metal second layer by either buttering a metal onto the synthetic resin material layer or by using a selective laser sintering process.

In an alternative method the first and second layers are provided as a sheath which is formed separately and slid onto the attachment portion in a distal to proximal direction.

This can be performed by preforming and bonding the two layers together by using additional PMMA bone cement.

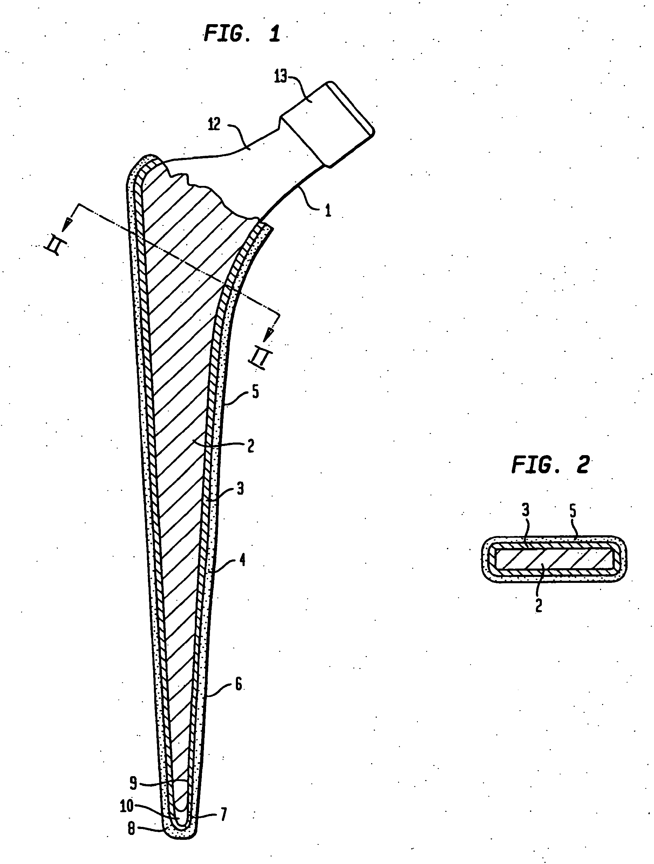

The invention can be performed in various ways but one embodiment will now be described by way of example and with reference to the accompanying drawings in which -
Figure 1 is a part-cross-sectional side elevation of a femoral prosthesis embodying the invention; and,
Figure 2 is a cross-sectional plan view on the line II-II shown in Figure 1.

As shown in the drawings, the invention is applied to a prosthetic implant in the form of a femoral prosthesis 1 which has an attachment portion for insertion into or attachment to a patient's bone without bone cement in the form of a stem 2. The outer surface of the stem 2 is smooth and may be polished and tapered on moving proximal to distal on the stem. The outer surface of the stem 2 is smooth and has a first layer 3 made of a synthetic resin material which in this example is polymethlymethacrylate.

A second layer made of a metallic material 4 is applied over the first layer 3 and the proximal part 5 of the second layer 4 is porous. The distal portion 6 (in the diaphysis of the femur) of the second metallic layer 4 is less porous or substantially non-porous in relation to the proximal part (in the epiphysis and metaphysic of the femur) 5.

With this construction the proximal part 5 of the second layer 4 which is porous provides for bone ingrowth and the distal portion 6 can either be less porous or substantially non-porous by providing it as a roughened surface to allow for bone ongrowth. Thus the maximum bone attachment is provided at the proximal end where the stem is proximally loaded. In the area where no distal locking is required the outer surface of the distal portion 6 could be relatively smooth.

Alternatively, the proximal part 5 of the second layer 4 can be roughened rather than being porous to allow for bone ongrowth and the distal portion 6 can be less roughened or substantially smooth to provide the same effect.

The second layer 4 can be made from titanium, titanium alloy or any other bio-compatible metal which contacts directly with the bone.

The distal end 7 of the first layer 3 and second layer 4 are formed as a cup 8, the inner surface of which is spaced away from the distal end 9 of the stem 2 to provide a void 10 when initially located in position. This void 10 can accept subsequent movement between the first layer 3 and the stem after fitting.

The thickness of the wall formed by the layer 3 and 4 can be between 1 mm and 3 mm and is preferably between 1.8 mm and 2.5 mm.

The first and second layers 3 and 4 can be applied in situ to the stem 2, for example, the plastic first layer can be cast into position on the stem with which it is to be used and this can then be temporarily fitted with a mechanism to create the distal void.

Alternatively, the first and second layers can he preformed as a separate sheath for attachment to the stem. Again, the plastic inner layer 3 can be cast on the stem or, alternatively, this inner layer can be moulded into the outer metallic second layer 4.

A sheath of this type can be made which is not preferentially matched with the intended femoral implant, for example, a sheath could be of a standard dimension which could be used with the existing implants.

During construction a first layer 3 can be applied to a stem 2 by dipping or a spraying process and the second metallic layer 4 can be applied, for example, by sputtering or any other layering process or it can be made by a laser melting process. Such as that disclosed in US Publications Nos. 20040191106 and 20060147332.

This will still allow the stem to move downwardly after fitting provided the surface of the stem has a suitable smooth surface.

Alternatively the first and second layers 3 and 4 can be preformed as a sheath by forming on a suitable former, for example, the stem with which it is intended to be matched.

If the proximal portion of the metallic layer 4 is porous it allows for boney ingrowth and firm fixation of the assembly into the bone and the solid or less porous distal portion 6 allows for bone ongrowth, this portion being fitted as an interference fit between the sheath and the surrounding bone when installed.

If desired the inner surface of the metal second layer 4 can be roughened to enhance the bonding to the outer surface of the synthetic first layer 3. Preferably this bonding can take place with the addition of a PMMA bone cement (not shown).

In the construction shown the femoral prosthesis has a neck 12 and a tapered spigot 13 to receive a prosthetic head bearing ball (not shown) of well-known type.

In the example described above the invention is applied to a femoral prosthesis but it can be equally well applied to any other prosthesis which has an attachment portion which is for insertion into or attachment to a patient's bone without bone cement.

If desired a layer of bio-active material, such as hydroxyapatite (not shown) can be applied to the outer surface of the second metallic material layer 4 to encourage bone ingrowth or ongrowth.

## Claims

1. A collarless prosthetic femoral implant (1) which has an attachment portion in the form of a stem (2) for insertion into or attachment to a patient's bone without bone cement, **characterised in that** the outer surface of said stem (2) is smooth and tapered from the proximal to the distal end, a first layer (3) made of a synthetic resin material extending over said stem (2) and which is not attached thereto to allow subsequent downward movement between the first layer (3) and the smooth stem (2) after fitting and a second layer (4) secured to said first layer (3) and made from a metallic material a first and proximal portion (5) thereof being porous or roughened to encourage bone ingrowth or ongrowth and a second and distal portion (6) thereof being less porous, substantially non-porous, or less roughened in relation to the first portion (5) or smooth.

2. A collarless prosthetic femoral implant as claimed in claim 1 **characterised in that** the first and second layers (3)(4) are applied in-situ to the attachment portion (2).

3. A collarless prosthetic femoral implant as claimed in claim 1 **characterised in that** the first and second layers (3)(4) are preformed as a separate sheath for attachment to said attachment portion.

4. A collarless prosthetic femoral implant plant as claimed in any one of preceding claims 1 to 3 **characterised in that** the metal layer (4) is titanium, titanium alloy or any other suitably bio-compatible metal.

5. A collarless prosthetic femoral implant as claimed in any one of preceding claims 1 to 4 **characterised in that** the metal layer (4) is in the form of a preformed shape.

6. A collarless prosthetic femoral implant as claimed in any one of preceding claims 1 to 4 **characterised in that** the metal layer (4) is formed by metallic sputtering.

7. A collarless prosthetic femoral implant as claimed in claim 6 **characterised in that** the metal layer (4) is formed by a laser melting process.

8. A collarless prosthetic femoral implant as claimed in any one of preceding claims 1 to 7 **characterised in that** the synthetic resin material is polymethylmethacrylate.

9. A collarless prosthetic femoral implant as claimed in any one of preceding claims 1 to 8 **characterised in that** the distal end of the first and second layers (3)(4) is formed as a cup (8), the inner surface of which is spaced away from the distal end (9) of the attachment portion (2) to provide a distal void (10) when initially located in position to accept subsequent movement between the first layer (3) and the attachment portion (2) after fitting.

10. A collarless prosthetic femoral implant as claimed in any one of the preceding claims 1 to 9 **characterised in that** the thickness of the wall formed by the two layers (3)(4) is between 1 mm and 3 mm.

11. A collarless prosthetic femoral implant as claimed in claim 10 **characterised in that** the thickness of the wall formed by the two layers (3)(4) is between 1.8 mm and 2.5 mm.

12. A collarless prosthetic femoral implant as claimed in any one of the preceding claims 1 to 11 **characterised in that** the inner surface of the metal second layer (4) is roughened to enhance the bonding to the outer surface of the synthetic resin first layer (3).

13. A collarless prosthetic femoral implant as claimed in claim 12 **characterised in that** the bonding takes place with the addition of a PMMA bone cement.

14. A method of forming the collarless prosthetic femoral implant set forth in claim 1 **characterised by** including attaching the first layer (3) of a synthetic resin material by dipping the attachment portion (2) into a liquid bath of material or by spraying the material onto the outer surface of the attachment portion (2), then applying the metal second layer (4) by either sputtering a metal onto the synthetic resin material layer (3) or by using a selective laser sintering process.

15. A method of forming the collarless prosthetic femoral implant set forth in claim 1 **characterised by** including providing the first and second layers(3)(4) as a sheath which is formed separately and slid onto the attachment portion (2) in a distal to proximal direction.

## Patentansprüche

1. Kragenloses Oberschenkelprothesenimplantat (1), welches einen Befestigungsabschnitt in Form eines Stiels (2) zur Einführung in oder Befestigung am Knochen eines Patienten ohne Knochenzement aufweist, **dadurch gekennzeichnet, dass** die äußere Oberfläche des Stiels (2) glatt ist und sich vom proximalen zum distalen Ende hin verjüngt, sich eine erste Schicht (3) aus einem synthetischen Harzmaterial über den Stiel (2) erstreckt und nicht daran befestigt ist, um so nach dem Einpassen die anschließende Abwärtsbewegung zwischen der ersten Schicht (3) und dem glatten Stiel (2) zu ermöglichen, und eine zweite Schicht (4) an der ersten Schicht (3) fixiert und aus einem metallischen Material gefertigt ist, wobei ein erster und proximaler Abschnitt (5) porös oder angeraut ist, was das Knocheneinwachsen oder -anwachsen fördert, und ein zweiter und distaler Abschnitt (6) in Bezug auf den ersten Abschnitt (5) weniger porös, im Wesentlichen nicht-porös oder weniger angeraut oder glatt ist.

2. Kragenloses Oberschenkelprothesenimplantat nach Anspruch 1, **dadurch gekennzeichnet, dass** die erste und zweite Schicht (3)(4) in-situ auf dem Befestigungsabschnitt (2) aufgetragen werden.

3. Kragenloses Oberschenkelprothesenimplantat nach Anspruch 1, **dadurch gekennzeichnet, dass** die erste und zweite Schicht (3)(4) als separate Hülle zur Befestigung an dem Befestigungsabschnitt vorgeformt sind.

4. Kragenloses Oberschenkelprothesenimplantat nach einem der vorstehenden Ansprüche 1 bis 3, **dadurch gekennzeichnet, dass** die Metallschicht (4) Titan, eine Titanlegierung oder irgendein anderes geeignetes biokompatibles Metall ist.

5. Kragenloses Oberschenkelprothesenimplantat nach einem der vorstehenden Ansprüche 1 bis 4, **dadurch gekennzeichnet, dass** die Metallschicht (4) in Form eines vorgeformten Formteils vorliegt.

6. Kragenloses Oberschenkelprothesenimplantat nach einem der vorstehenden Ansprüche 1 bis 4, **dadurch gekennzeichnet, dass** die Metallschicht (4) durch Metallsputtern gebildet wird.

7. Kragenloses Oberschenkelprothesenimplantat nach Anspruch 6, **dadurch gekennzeichnet, dass** die Metallschicht (4) durch ein Laserschmelzverfahren gebildet wird.

8. Kragenloses Oberschenkelprothesenimplantat nach einem der vorstehenden Ansprüche 1 bis 7, **dadurch gekennzeichnet, dass** das synthetische Harzmaterial Polymethylmethacrylat ist.

9. Kragenloses Oberschenkelprothesenimplantat nach einem der vorstehenden Ansprüche 1 bis 8, **dadurch gekennzeichnet, dass** das distale Ende der ersten und zweiten Schicht (3)(4) als eine Pfanne (8) geformt ist, deren innere Oberfläche vom distalen Ende (9) des Befestigungsabschnittes (2) beabstandet angeordnet ist, wodurch bei der Ausgangspositionierung ein distaler Hohlraum (10) bereitgestellt wird, der nach der Einpassung die anschließende Bewegung zwischen der ersten Schicht (3) und dem Befestigungsabschnitt (2) ermöglicht.

10. Kragenloses Oberschenkelprothesenimplantat nach einem der vorstehenden Ansprüche 1 bis 9, **dadurch gekennzeichnet, dass** die Dicke der durch die beiden Schichten (3)(4) gebildeten Wand zwischen 1 mm und 3 mm liegt.

11. Kragenloses Oberschenkelprothesenimplantat nach Anspruch 10, **dadurch gekennzeichnet, dass** die Dicke der durch die beiden Schichten (3)(4) gebildeten Wand zwischen 1,8 mm und 2,5 mm liegt.

12. Kragenloses Oberschenkelprothesenimplantat nach einem der vorstehenden Ansprüche 1 bis 11, **dadurch gekennzeichnet, dass** die innere Oberfläche der zweiten Metallschicht (4) angeraut ist, um so die Bindung an die äußere Oberfläche der ersten Schicht (3) aus synthetischem Harz zu verstärken.

13. Kragenloses Oberschenkelprothesenimplantat nach Anspruch 12, **dadurch gekennzeichnet, dass** die Bindung durch die Zugabe eines PMMA-Knochenzements stattfindet.

14. Verfahren zur Formung des kragenlosen Oberschenkelprothesenimplantats nach Anspruch 1, **dadurch gekennzeichnet, dass** es das Befestigen der ersten Schicht (3) aus einem synthetischen Harzmaterial durch Eintauchen des Befestigungsabschnittes (2) in ein Flüssigkeitsbad aus dem Material oder durch Sprühen des Materials auf die äußere Oberfläche des Befestigungsabschnittes (2), dann das Auftragen der zweiten Metallschicht (4) durch entweder Sputtern eines Metalls auf die Schicht (3) aus synthetischem Harzmaterial oder durch Verwendung eines selektiven Lasersinterverfahrens umfasst.

15. Verfahren zur Formung des kragenlosen Oberschenkelprothesenimplantats nach Anspruch 1, **dadurch gekennzeichnet, dass** es das Bereitstellen der ersten und zweiten Schicht (3)(4) als eine Hülle umfasst, die separat geformt und von distaler zu proximaler Richtung auf den Befestigungsabschnitt (2) geschoben wird.

## Revendications

1. Implant fémoral prothétique sans collerette (1) qui comporte une partie de fixation sous la forme d'une tige (2) pour l'insertion dans l'os d'un patient ou la fixation à celui-ci sans ciment à os, **caractérisé en ce que** la surface extérieure de ladite tige (2) est lisse et effilée de l'extrémité proximale à l'extrémité distale, une première couche (3) réalisée en un matériau en résine synthétique s'étendant sur ladite tige (2) et n'étant pas fixée à celle-ci, de façon à permettre un mouvement ultérieur vers le bas entre la première couche (3) et la tige lisse (2) après la mise en place, et une deuxième couche (4) étant fixée à ladite première couche (3) et réalisée un matériau métallique, une première extrémité, proximale, (5) de celle-ci étant poreuse ou rugueuse de façon à encourager la croissance osseuse interne ou de recouvrement, et une deuxième partie, distale, (6) de celle-ci étant moins poreuse, sensiblement non poreuse ou moins rugueuse que la première partie (5), ou lisse.

2. Implant fémoral prothétique sans collerette selon la revendication 1, **caractérisé en ce que** les première et deuxième couches (3) (4) sont appliquées in situ à la partie de fixation (2).

3. Implant fémoral prothétique sans collerette selon la revendication 1, **caractérisé en ce que** les première et deuxième couches (3) (4) sont préformées sous la forme d'un manchon séparé pour la fixation à ladite partie de fixation.

4. Implant fémoral prothétique sans collerette selon l'une quelconque des revendications 1 à 3 précédentes, **caractérisé en ce que** la couche métallique (4) est en titane, en alliage de titane ou en un quelconque autre métal bio-compatible approprié.

5. Implant fémoral prothétique sans collerette selon l'une quelconque des revendications 1 à 4 précédentes, **caractérisé en ce que** la couche métallique (4) se présente sous la forme d'une forme préformée.

6. Implant fémoral prothétique sans collerette selon l'une quelconque des revendications 1 à 4 précédentes, **caractérisé en ce que** la couche métallique (4) est réalisée par pulvérisation cathodique métallique.

7. Implant fémoral prothétique sans collerette selon la revendication 6, **caractérisé en ce que** la couche métallique (4) est formée par un processus de fusion au laser.

8. Implant fémoral prothétique sans collerette selon l'une quelconque des revendications 1 à 7 précédentes, **caractérisé en ce que** le matériau en résine synthétique est du polyméthacrylate de méthyle.

9. Implant fémoral prothétique sans collerette selon l'une quelconque des revendications 1 à 8 précédentes, **caractérisé en ce que** l'extrémité distale des première et deuxième couches (3) (4) est formée sous la forme d'une coupelle (8) dont la surface intérieure est espacée de l'extrémité distale (9) de la partie de fixation (2) de façon à constituer un vide distal (10) lors de la disposition initiale en position, afin d'admettre un mouvement ultérieur entre la première couche (3) et la partie de fixation (2) après disposition.

10. Implant fémoral prothétique sans collerette selon l'une quelconque des revendications 1 à 9 précédentes, **caractérisé en ce que** l'épaisseur de la paroi formée par les deux couches (3) (4) est comprise entre 1 mm et 3 mm.

11. Implant fémoral prothétique sans collerette selon la revendication 10, **caractérisé en ce que** l'épaisseur de la paroi formée par les deux couches (3) (4) est comprise entre 1,8 mm et 2,5 mm.

12. Implant fémoral prothétique sans collerette selon l'une quelconque des revendications 1 à 11 précédentes, **caractérisé en ce que** la surface intérieure de la deuxième couche métallique (4) est rendue rugueuse, de façon à améliorer l'adhérence avec la surface extérieure de la première couche en résine synthétique (3).

13. Implant fémoral prothétique sans collerette selon la revendication 12, **caractérisé en ce que** l'adhérence est produite avec l'addition d'un ciment à os au polyméthacrylate de méthyle.

14. Procédé de formation de l'implant fémoral prothétique sans collerette selon la revendication 1, **caractérisé en ce qu'**il comprend la fixation de la première couche (3) en un matériau en résine synthétique par trempage de la partie de fixation (2) dans un bain liquide de matériau ou par pulvérisation du matériau sur la surface extérieure de la partie de fixation (2), puis l'application de la deuxième couche métallique (4) soit par pulvérisation cathodique d'un métal sur la couche de matériau en résine synthétique (3) soit par utilisation d'un processus de frittage au laser sélectif.

15. Procédé de formation de l'implant fémoral prothétique sans collerette selon la revendication 1, **caractérisé en ce qu'**il comprend la réalisation des première et deuxième couches (3) (4) sous la forme d'un manchon qui est formé séparément et que l'on fait glisser sur la partie de fixation (2) dans une direction distale-proximale.
